# EUROPEAN PATENT APPLICATION

(11) **EP 3 964 264 A1**
(43) Date of publication of application: **09.03.2022**
(21) Application number: 20799485.6
(22) Date of filing: 24.04.2020
(51) Int. Cl.: A61P 7/06, A61P 13/12, A61K 31/194

(54) **NOVEL PHARMACEUTICAL COMPOSITION**

(30) Priority: 28.04.2019 JP 2019086945; 27.06.2019 JP 2019119297
(71) Applicant: Tohoku University, Sendai-shi, Miyagi 980-8577 (JP); Nippon Chemiphar Co., Ltd., Tokyo 101-0032 (JP)
(72) Inventor: ABE Michiaki, Sendai-shi, Miyagi 980-8577 (JP); KOSHIBA Seizo, Sendai-shi, Miyagi 980-8577 (JP); NISHIOKA Koichiro, Tokyo 101-0032 (JP); KAWAGUCHI Kazuhiko, Tokyo 101-0032 (JP); YAMASAKI Satomi, Tokyo 101-0032 (JP); TERANAKA Yasuyuki, Tokyo 101-0032 (JP)
(74) Representative: V.O.
(86) International application number: PCT/JP2020/017690
(87) International publication number: WO 2020/222302

(57) **Abstract**

The present invention relates to a pharmaceutical composition for treating or preventing acidosis in chronic kidney disease with anemia, the pharmaceutical composition containing citric acid, a pharmaceutically acceptable salt of citric acid, a hydrate thereof, or a mixture thereof, in which the pharmaceutical composition is in the form of a tablet.

## Description

### Technical Field

The present invention relates to a novel pharmaceutical composition containing citric acid, a pharmaceutically acceptable salt of citric acid, a hydrate thereof, or a mixture thereof. More specifically, the present invention relates to a pharmaceutical composition for suppressing acidosis in a chronic kidney disease patient with anemia, suppressing anemia, suppressing a decrease in blood iron concentration, or suppressing a decrease in blood ferritin concentration.

This application claims priority based on Japanese Patent Application No. 2019-86945 filed in Japan on April 28, 2019 and Japanese Patent Application No. 2019-119297 filed in Japan on June 27, 2019, the content of which is incorporated herein by reference.

### Background Art

The number of end-stage kidney disease (ESKD) patients requiring dialysis or transplantation is increasing worldwide. In Japan, the number of ESKD patients requiring dialysis or transplantation is increasing as well, and the number of dialysis patients reached 320,000 at the end of 2014.

Chronic kidney disease (CKD) is recognized as a predecessor of this ESKD. CKD is a concept including chronic kidney disease regardless of a primary disease, and is a concept including all pathological conditions that a renal function represented by glomerular filtration rate (GFR) decreases or findings suggesting renal disorder persist chronically (three months or more). There is not only a risk that CKD may progress to ESKD but also a strong risk that CKD may develop cardiovascular disease (CVD) or the like. Therefore, it is very important to detect CKD at an early stage and provide appropriate treatment. Many CKD treatment methods have been established so far, but they are still inadequate, and development of a renal protective agent is required.

A patient with progressed CKD has a low blood bicarbonate ion (HCO₃⁻) concentration and develops metabolic acidosis. Therefore, an alkalizing agent such as sodium bicarbonate or a citric acid formulation is administered to the patient with progressed CKD. It has been reported that progress of CKD is suppressed by administration of sodium bicarbonate, which is an alkalizing agent (Non Patent Literature 1). In addition, it has been reported that oral administration of sodium bicarbonate suppresses tubule cell damage caused by acidic urine in a nephrotic animal model caused by protein overload (Non Patent Literature 2). It is also known that administration of an alkalizing agent to an early-stage CKD patient suppresses progression of renal disorder and reduces a blood uremic substance concentration (Patent Literatures 1 and 2).

Meanwhile, a CKD patient causes anemia mainly because of erythropoietin deficiency. In addition, there are many factors that affect iron metabolism, and the blood iron concentration and the ferritin concentration decrease. However, it is not known that administration of an alkalizing agent to a CKD patient can suppress a decrease in blood iron concentration and a decrease in blood ferritin concentration.

### Citation List

### Patent Literature

Patent Literature 1: WO 2018/193648 A
Patent Literature 2: WO 2018/193752 A

### Non Patent Literature

Non Patent Literature 1: Brito-Ashurst, I.D., et al.: Bicarbonate supplementation slows progression of CKD and improves nutritional status. J. Am. Soc. Nephrol., 20: 2075-2084, 2009.
Non Patent Literature 2: Souma T., et al.: Luminal alkalinization attenuates proteinuria-induced oxidative damage in proximal tubular cells. J. Am. Soc. Nephrol., 22: 635-648, 2011.

### Summary of Invention

### Technical Problem

One object of the present invention is to provide a drug useful for treating or preventing acidosis in a chronic kidney disease patient with anemia, the drug containing citric acid, a pharmaceutically acceptable salt of citric acid, a hydrate thereof, or a mixture thereof. Another object of the present invention is to provide a drug useful for suppressing anemia (for example, suppressing renal anemia or suppressing iron deficiency anemia). Another object of the present invention is to provide a drug useful for suppressing a decrease in blood iron concentration or suppressing a decrease in blood ferritin concentration. Another object of the present invention is to provide a food for suppressing acidosis in a chronic kidney disease patient with anemia, suppressing anemia (for example, suppressing renal anemia or suppressing iron deficiency anemia), suppressing a decrease in blood iron concentration, or suppressing a decrease in blood ferritin concentration.

### Solution to Problem

As a result of intensive studies to achieve the above problems, the present inventors have found that a composition containing citric acid, a pharmaceutically acceptable salt of citric acid, a hydrate thereof, or a mixture thereof is useful for suppressing a decrease in blood iron concentration or a decrease in blood ferritin concentration, and that the drug is useful for suppressing acidosis in a chronic kidney disease patient with anemia or an acute kidney disease patient with anemia or suppressing anemia (for example, suppressing renal anemia or suppressing iron deficiency anemia), and have completed the present invention.

In one aspect, the present invention provides an anemia-suppressing pharmaceutical composition containing citric acid, a pharmaceutically acceptable salt of citric acid, a hydrate thereof, or a mixture thereof.

In one aspect, the present invention provides a pharmaceutical composition for suppressing acidosis in a chronic kidney disease patient with anemia or an acute kidney disease patient with anemia, the pharmaceutical composition containing citric acid, a pharmaceutically acceptable salt of citric acid, a hydrate thereof, or a mixture thereof.

In one aspect, the present invention provides a pharmaceutical composition for suppressing a decrease in blood iron concentration as compared with sodium bicarbonate or suppressing a decrease in blood ferritin concentration as compared with sodium bicarbonate, the pharmaceutical composition containing citric acid, a pharmaceutically acceptable salt of citric acid, a hydrate thereof, or a mixture thereof.

In one aspect, the present invention provides a food useful for suppressing acidosis in a chronic kidney disease patient with anemia or an acute kidney disease patient with anemia or suppressing anemia, the food containing citric acid, a pharmaceutically acceptable salt of citric acid, a hydrate thereof, or a mixture thereof.

In one aspect, the present invention provides a food useful for suppressing a decrease in blood iron concentration, as compared with sodium bicarbonate, or suppressing a decrease in blood ferritin concentration, as compared with sodium bicarbonate, the food containing citric acid, a pharmaceutically acceptable salt of citric acid, a hydrate thereof, or a mixture thereof.

### Advantageous Effects of Invention

A pharmaceutical composition, a food composition, and the like, provided by the present invention can suppress a decrease in blood iron concentration, as compared with sodium bicarbonate, or can suppress a decrease in blood ferritin concentration, as compared with sodium bicarbonate, in a mammal.

The pharmaceutical composition, the food composition, and the like, provided by the present invention can suppress acidosis in a chronic kidney disease patient with anemia or an acute kidney disease patient with anemia or can suppress anemia in a mammal.

### Description of Embodiments

### 1. Pharmaceutical composition

A pharmaceutical composition provided by the present invention may contain citric acid, a pharmaceutically acceptable salt of citric acid, a hydrate thereof, or a mixture thereof as an active ingredient.

Examples of pharmaceutically acceptable salt of citric acid include an alkali metal citrate. Examples of the alkali metal citrate include tripotassium citrate (hereinafter referred to as potassium citrate) and trisodium citrate (hereinafter referred to as sodium citrate), which may be hydrates such as stable potassium citrate monohydrate (C₆H₅K₃O₇•H₂O) and stable sodium citrate dihydrate (C₆H₅Na₃O₇•2H₂O), respectively. Examples of citric acid include, but are not limited to, anhydrous citric acid.

Examples of a preferable active ingredient contained in the pharmaceutical composition provided by the present invention include sodium citrate, potassium citrate, a hydrate thereof, and a mixture thereof, which may be, for example, a mixture of potassium citrate monohydrate (C₆H₅K₃O₇•H₂O) and sodium citrate dihydrate (C₆H₅Na₃O₇•2H₂O). The mixing ratio between potassium citrate monohydrate (C₆H₅K₃O₇•H₂O) and sodium citrate dihydrate (C₆H₅Na₃O₇•2H₂O) can be appropriately set by a person skilled in the art. For example, the molar ratio of potassium citrate monohydrate : sodium citrate dihydrate can be 1:0.01 to 100. The molar ratio between potassium citrate (for example, potassium citrate monohydrate) and sodium citrate (for example, sodium citrate dihydrate) can be appropriately set by a person skilled in the art, and may be, for example, 0.85:1.15 to 1.15:0.85, 0.90:1.10 to 1.10:0.90, 0.95:1.05 to 1.05:0.95, or 0.99:1.01 to 1.01:0.99, and is preferably 1:1.

Other examples of the active ingredient contained in the pharmaceutical composition provided by the present invention include sodium citrate and a hydrate thereof, and may be, for example, sodium citrate dihydrate (C₆H₅Na₃O₇•2H₂O).

Other examples of the active ingredient contained in the pharmaceutical composition provided by the present invention include potassium citrate and a hydrate thereof, and may be, for example, potassium citrate monohydrate (C₆H₅K₃O₇•H₂O).

In one embodiment, the active ingredient contained in the pharmaceutical composition of the present invention may contain a mixture of sodium citrate or a hydrate thereof and potassium citrate or a hydrate thereof.

In one embodiment, the active ingredient contained in the pharmaceutical composition of the present invention may be a mixture of potassium citrate, sodium citrate, and citric acid (for example, anhydrous citric acid). In this case, the mixing ratio (molar ratio) among citric acid (for example, anhydrous citric acid), potassium citrate, and sodium citrate can be appropriately set by a person skilled in the art, and may be, for example, 1:1.7 to 2.3:1.7 to 2.3, 1:1.9 to 2.1:1.9 to 2.1, or 1:1.95 to 2.05:1.95 to 2.05, and is preferably 1:2:2.

In one embodiment, the active ingredient contained in the pharmaceutical composition of the present invention may be a mixture of potassium citrate monohydrate (C₆H₅K₃O₇•H₂O), sodium citrate dihydrate (C₆H₅Na₃O₇•2H₂O), and anhydrous citric acid. In this case, the mixing ratio (molar ratio) among anhydrous citric acid, potassium citrate monohydrate (C₆H₅K₃O₇•H₂O), and sodium citrate dihydrate (C₆H₅Na₃O₇•2H₂O) can be appropriately set by a person skilled in the art, and may be, for example, 1:1.7 to 2.3:1.7 to 2.3, 1:1.9 to 2.1:1.9 to 2.1, or 1:1.95 to 2.05:1.95 to 2.05, and is preferably 1:2:2.

In another embodiment, the active ingredient contained in the pharmaceutical composition of the present invention may contain only a mixture of sodium citrate or a hydrate thereof and potassium citrate or a hydrate thereof.

Here, when the weight of citric acid, a pharmaceutically acceptable salt of citric acid, a hydrate thereof, or a mixture thereof (for example, potassium citrate monohydrate (C₆H₅K₃O₇•H₂O) and sodium citrate dihydrate (C₆H₅Na₃O₇•2H₂O)) is referred to, the weight can be a dry weight.

The pharmaceutical composition provided by the present invention can also be used for treating or preventing chronic kidney disease or acute kidney disease.

For example, administration of the pharmaceutical composition provided by the present invention suppresses anemia (for example, renal anemia or iron deficiency anemia).

In addition, for example, administration of the pharmaceutical composition provided by the present invention suppresses a decrease in serum iron concentration and suppresses a decrease in serum ferritin concentration, as compared with administration of sodium bicarbonate. Therefore, the pharmaceutical composition provided by the present invention is useful for treating or preventing acidosis in a chronic kidney disease patient with anemia suppressed, treating or preventing acidosis in a chronic kidney disease patient with anemia, treating or preventing acidosis in an acute kidney disease patient with anemia suppressed, or treating or preventing acidosis in an acute kidney disease patient with anemia.

In addition, for example, administration of the pharmaceutical composition provided by the present invention suppresses a decrease in blood iron concentration or a decrease in blood ferritin concentration.

Here, "suppression" is a concept including stopping or slowing exacerbation or progression of a symptom, condition, or disease, and performance for this purpose, and including improving the symptom, condition, or disease, or performance for this purpose. Here, "improvement" is a concept including bringing a "pathological" or "abnormal" symptom, condition, or disease closer to a "healthy" or "normal" condition, or performance for this purpose, and bringing the "pathological" or "abnormal" symptom, condition, or disease to a "healthy" or "normal" condition, or performance for this purpose. Therefore, in one embodiment, "improvement" includes that a numerical value that is an index of a "pathological" or "abnormal" symptom or condition becomes smaller or larger to approach, or become, a normal value according to the "improvement". The "exacerbation or progression of a symptom, condition, or disease" includes exacerbation or progression of a "pathological" or "abnormal" symptom, condition, or disease, and exacerbation or progression from a "healthy" or "normal" condition to a "pathological" or "abnormal" symptom, condition, or disease,. In one embodiment, "suppression" is to stop or slow exacerbation or progression of a symptom, condition, or disease, or performance for this purpose. In another embodiment, "suppression" is to stop or slow exacerbation or progression of a symptom, condition, or disease.

Here, "healthy" indicates a condition free of acute or chronic disease or disorder, and "normal" indicates a condition normally expressed by a healthy subject.

Here, the symptom, condition, or disease before administration of the pharmaceutical composition provided by the present invention is compared with that after administration thereof. Alternatively, the symptom, condition, or disease when the pharmaceutical composition provided by the present invention is administered is compared with that when a control or a placebo is administered.

Here, "treatment" is a concept including eliminating, completely recovering, curing, or ameliorating a "pathological" or "abnormal" symptom, condition, or disease, and performance for this purpose, including "suppressing" exacerbation of the "pathological" or "abnormal" symptom, condition, or disease, and performance for this purpose, and also including "improvement". Here, "suppression" and "improvement" have the above meanings.

In one embodiment, "treatment" is to eliminate, completely recover, cure, or ameliorate a "pathological" or "abnormal" symptom, condition, or disease, and performance for this purpose. In another embodiment, "treatment" is to eliminate, completely recover, cure, or ameliorate a "pathological" or "abnormal" symptom, condition, or disease.

Here, "prevention" is a concept including preventing onset of a "pathological" or "abnormal" symptom, condition, or disease, and performance for this purpose.

In one embodiment, "anemia" can be evaluated by a lower blood iron concentration as compared with a "healthy" or "normal" condition. Therefore, in one embodiment, "suppression of anemia" can be evaluated by a fact that a decrease in blood iron concentration or blood ferritin concentration after administration of the pharmaceutical composition provided by the present invention is suppressed, as compared with the blood iron concentration or the blood ferritin concentration before administration thereof, or a fact that a decrease in blood iron concentration or blood ferritin concentration is suppressed by administration of the pharmaceutical composition provided by the present invention, as compared with placebo administration or a control.

In one embodiment, "suppression of anemia" can be evaluated by a fact that serum iron amounts (concentration; µg/dL) in 6 weeks, 12 weeks, and 24 weeks after administration of the pharmaceutical composition provided by the present invention are 100% or more and less than 120%, preferably 105% or more and 115% or less and, more preferably, 105% or more and 110% or less, with respect to serum iron amounts 6 weeks, 12 weeks, and 24 weeks after administration of a control, respectively.

In one embodiment, "suppression of anemia" can be evaluated by a fact that each of serum iron amounts (concentration; µg/dL) in 6 weeks, 12 weeks, and 24 weeks after administration of the pharmaceutical composition provided by the present invention is 100% or more and less than 120%, preferably 100% or more and 115% or less and, more preferably, 100% or more and 110% or less, with respect to a serum iron amount before administration thereof.

In one embodiment, "suppression of anemia" can be evaluated by a fact that each of serum iron amounts (concentration; µg/dL) 6 weeks, 12 weeks, and 24 weeks after administration of the pharmaceutical composition provided by the present invention increases by 0 µg/dL or more and 20 µg/dL or less, preferably 0 µg/dL or more and 15 µg/dL or less, more preferably 5 µg/dL or more and 10 µg/dL or less, as compared with a serum iron amount before administration thereof.

In one embodiment, "suppression of anemia" can be evaluated by a fact that a serum ferritin concentration (ng/mL) 24 weeks after administration of the pharmaceutical composition provided by the present invention is 100% or more and less than 200%, preferably 100% or more and 150% or less and, more preferably, 120% or more and 150% or less, with respect to a serum ferritin concentration 24 weeks after administration of a control.

In one embodiment, "suppression of anemia" can be evaluated by a fact that a serum ferritin concentration (ng/mL) 24 weeks after administration of the pharmaceutical composition provided by the present invention is 80% or more and less than 160%, preferably 80% or more and 120% or less and, more preferably, 90% or more and 110% or less, with respect to a serum ferritin concentration before administration thereof. In one embodiment, "suppression of anemia" can be evaluated by a fact that a serum ferritin concentration (ng/mL) 24 weeks after administration of the pharmaceutical composition provided by the present invention increases by -20 ng/mL or more and 20 ng/mL or less, preferably -15 ng/mL or more and 15 ng/mL or less, more preferably -12 ng/mL or more and 12 ng/mL or less, with respect to a serum ferritin concentration before administration thereof.

In one embodiment, "suppression of a decrease in blood iron concentration" or "suppression of a decrease in blood ferritin concentration" can be evaluated by a fact that an increase or a decrease in concentration of each ingredient after administration of the pharmaceutical composition provided by the present invention is suppressed, as compared with a concentration of each ingredient before administration thereof, or a fact that an increase or a decrease in concentration of each ingredient is suppressed by administration of the pharmaceutical composition provided by the present invention, as compared with placebo administration or a control.

Here, "early morning urine" indicates first urine after waking up, and "spot urine" indicates urine other than the "early morning urine".

The pharmaceutical composition provided by the present invention is orally or parenterally administered to a human or another mammal. Examples of the parenteral administration include intravenous administration, subcutaneous administration, intramuscular administration, intraarticular administration, transmucosal administration, transdermal administration, nasal administration, rectal administration, intrathecal administration, intraperitoneal administration, and local administration.

The pharmaceutical composition provided by the present invention may be prepared by using citric acid, a pharmaceutically acceptable salt of citric acid, a hydrate thereof, or a mixture thereof, which is an active ingredient, as it is, or by mixing citric acid, a pharmaceutically acceptable salt of citric acid, a hydrate thereof, or a mixture thereof with a pharmaceutically acceptable carrier such as an excipient (for example, lactose, D-mannitol, crystalline cellulose, or glucose), a binder (for example, hydroxypropyl cellulose (HPC), gelatin, or polyvinylpyrrolidone (PVP)), a lubricant (for example, magnesium stearate or talc), a disintegrant (for example, starch or carboxymethyl cellulose calcium (CMC-Ca)), or a diluent (for example, injection water or physiological saline), and another additive if necessary (for example, a pH regulator, a surfactant, a solubilizer, a preservative, an emulsifier, a tonicity agent, or a stabilizer), and can be a formulation in a form of a tablet, a capsule, a suspending agent, an injection, a suppository, or the like. For example, in order to prepare the pharmaceutical composition in a form of a tablet, citric acid, a pharmaceutically acceptable salt of citric acid, a hydrate thereof, or a mixture thereof, which is an active ingredient, may be mixed with an excipient (for example, lactose, D-mannitol, crystalline cellulose, or glucose), a disintegrant (for example, starch or carboxymethyl cellulose calcium (CMC-Ca)), a binder (for example, hydroxypropyl cellulose (HPC), gelatin, or polyvinylpyrrolidone (PVP)), a lubricant (for example, magnesium stearate or talc), or the like, and formulated.

In one embodiment, the pharmaceutical composition provided by the present invention is in a form of a tablet. The tablet provided by the present invention may contain, in addition to citric acid, a pharmaceutically acceptable salt of citric acid, a hydrate thereof, or a mixture thereof (for example, potassium citrate or a hydrate thereof; sodium citrate or a hydrate thereof; a mixture of potassium citrate monohydrate and sodium citrate dihydrate; or sodium bicarbonate), which is an active ingredient, a pharmaceutically acceptable additive commonly used in the pharmaceutical field. Examples of such an additive include an excipient, a binder, a disintegrant, a fluidizer, a flavoring agent, a lubricant, a pH regulator, a surfactant, a stabilizer, and a fragrance.

The content of citric acid, a pharmaceutically acceptable salt of citric acid, a hydrate thereof, or a mixture thereof, which is an active ingredient in the tablet provided by the present invention, may be 10 to 95% by weight, preferably 30 to 90% by weight and, more preferably, 60 to 85% by weight, with respect to the tablet.

The pharmaceutical composition provided by the present invention can be manufactured by a method known in the pharmaceutical field.

In one embodiment, the hardness of a tablet to be obtained can be 10 to 200 N, and preferably 30 to 150 N.

The content of citric acid, a pharmaceutically acceptable salt of citric acid, a hydrate thereof, or a mixture thereof, which is an active ingredient in the pharmaceutical composition provided by the present invention, can be appropriately set.

In one embodiment, the content of citric acid, a pharmaceutically acceptable salt of citric acid, a hydrate thereof, or a mixture thereof, which is an active ingredient in the pharmaceutical composition provided by the present invention, may be set such that a dose of citric acid, a pharmaceutically acceptable salt of citric acid, a hydrate thereof, or a mixture thereof, which is an active ingredient, is equal to or smaller than an amount to improve acidic urine in gout or hyperuricemia by being administered to a human, for example, such that the dose of citric acid, a pharmaceutically acceptable salt of citric acid, a hydrate thereof, or a mixture thereof is 1 to 50% or 10 to 20% of a daily dose approved in Japan for improving acidic urine in gout or hyperuricemia (for example, in a case of citric acid formulation: two tablets each containing 231.5 mg of potassium citrate (C₆H₅K₃O₇•H₂O) and 195.0 mg of sodium citrate hydrate (C₆H₅Na₃O₇•2H₂O) are orally administered three times a day).

In one embodiment, the pharmaceutical composition provided by the present invention is in a form of a tablet, and may contain, per tablet, 10 mg to 1 g, preferably 100 mg to 500 mg, more preferably 400 mg to 500 mg, of potassium citrate monohydrate or sodium citrate dihydrate.

In one embodiment, the pharmaceutical composition provided by the present invention is in a form of a tablet, and may contain, per tablet, potassium citrate monohydrate and sodium citrate dihydrate as active ingredients in each amount of 10 mg to 300 mg for a total of 20 mg to 600 mg, preferably in each amount of 150 to 250 mg for a total of 400 to 500 mg, more preferably in each amount of 190 to 240 mg for a total of 400 to 450 mg.

In one embodiment, the pharmaceutical composition provided by the present invention is in a form of a tablet, may contain 231.5 mg of potassium citrate monohydrate and 195.0 mg of sodium citrate dihydrate as active ingredients, and may contain anhydrous citric acid, crystalline cellulose, partially pregelatinized starch, hydroxypropyl cellulose, magnesium stearate, hypromellose, macrogol 6000, titanium oxide, and carnauba wax as additives. In this embodiment, the content of anhydrous citric acid may be 72.5 mg.

In one embodiment, a tablet containing 231.5 mg of potassium citrate monohydrate and 195.0 mg of sodium citrate dihydrate may be used as one administration unit.

Here, "administration unit" indicates a unit of a formulation, and "one administration unit" indicates a minimum unit of a formulation. Therefore, for example, in a case of a tablet, an administration unit is each tablet, and one administration unit indicates one tablet. In a case of an injection, an administration unit is an injection contained in a sealed container such as an ampoule or a vial, and one administration unit indicates an injection contained in one sealed container such as an ampoule or a vial.

When the pharmaceutical composition provided by the present invention is administered to a human or another mammal, one or more of the above-described administration units may be administered at a time, or the above-described one administration unit may be divided into portions and administered.

The dose of citric acid, a pharmaceutically acceptable salt of citric acid, a hydrate thereof, or a mixture thereof, which is an active ingredient, is appropriately determined according to the type of citric acid, a pharmaceutically acceptable salt of citric acid, a hydrate thereof, or a mixture thereof, which is an active ingredient, an administration method, age of an administration subject, weight thereof, sex thereof, a symptom thereof, sensitivity thereof to a drug, and the like, but the dose may be adjusted according to the situation of improvement of a symptom.

In one embodiment, when a mixture of potassium citrate monohydrate and sodium citrate dihydrate as an active ingredient is orally administered to a human, a half of a daily dose approved in Japan for improving acidic urine in gout and hyperuricemia (for example, in the case of citric acid formulation: two tablets each containing 231.5 mg of potassium citrate (C₆H₅K₃O₇•H₂O) and 195.0 mg of sodium citrate hydrate (C₆H₅Na₃O₇•2H₂O) are orally administered three times a day) may be administered a day.

In one embodiment, when a mixture of potassium citrate monohydrate and sodium citrate dihydrate as an active ingredient is orally administered to a human, a daily dose approved in Japan for improving acidic urine in gout and hyperuricemia (for example, in the case of citric acid formulation: two tablets each containing 231.5 mg of potassium citrate (C₆H₅K₃O₇•H₂O) and 195.0 mg of sodium citrate hydrate (C₆H₅Na₃O₇•H₂O) are orally administered three times a day) may be administered a day.

In one embodiment, when a mixture of potassium citrate monohydrate and sodium citrate dihydrate as an active ingredient is orally administered to a human, a half of a daily dose approved in Japan for improving acidic urine in gout and hyperuricemia (for example, in the case of citric acid formulation: two tablets each containing 231.5 mg of potassium citrate (C₆H₅K₃O₇•H₂O) and 195.0 mg of sodium citrate hydrate (C₆H₅Na₃O₇•2H₂O) are orally administered three times a day) may be administered a day in the beginning, and then the dose may be increased up to the daily dose approved in Japan for improving acidic urine in gout and hyperuricemia.

In one embodiment, the dose of citric acid, a pharmaceutically acceptable salt of citric acid, a hydrate thereof, or a mixture thereof, which is an active ingredient, may be such that the pH of human urine (for example, early morning urine) is pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to 6.8, pH 6.2 to pH 6.5, or pH 6.5 to 6.8 by oral administration of citric acid, a pharmaceutically acceptable salt of citric acid, a hydrate thereof, or a mixture thereof, which is an active ingredient.

In one embodiment, the dose of citric acid, a pharmaceutically acceptable salt of citric acid, a hydrate thereof, or a mixture thereof, which is an active ingredient, may be such that the pH of human urine (for example, early morning urine) is, 6 weeks, 12 weeks, or 24 weeks after administration, pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to 6.8, pH 6.2 to pH 6.5, or pH 6.5 to 6.8 by oral administration of citric acid, a pharmaceutically acceptable salt of citric acid, a hydrate thereof, or a mixture thereof, which is an active ingredient.

In one embodiment, when a mixture of potassium citrate monohydrate and sodium citrate dihydrate as an active ingredient is orally administered to a human, potassium citrate monohydrate and sodium citrate dihydrate may be administered each in an amount of 0.1 to 5 g/day for a total of 0.2 to 10 g/day, each in an amount of 0.1 to 3 g/day for a total of 0.2 to 6 g/day, or each in an amount of 0.5 to 3 g/day for a total of 1 to 6 g/day, preferably each in an amount of 0.5 to 1.5 g/day for a total of 1 to 3 g/day, each in an amount of 1 to 1.5 g/day for a total of 2 to 3 g/day, or each in an amount of 0.5 to 1 g/day for a total of 1 to 2 g/day, in which the daily dose may be divided into one to five portions, preferably three portions, to be administered a day.

In one embodiment, when potassium citrate monohydrate or sodium citrate dihydrate as an active ingredient is orally administered to a human, potassium citrate monohydrate or sodium citrate dihydrate may be administered in an amount of 1 to 10 g/day, 1 to 6 g/day, 2 to 5.5 g/day, 1 to 3 g/day, 2 to 3 g/day, or 1 to 1.5 g/day, in which the daily dose may be divided into one to five portions, preferably three portions, to be administered a day.

In one embodiment, citric acid, a pharmaceutically acceptable salt of citric acid, a hydrate thereof, or a mixture thereof, which is an active ingredient, may be administered over a long period of time, and for example, is administered for one week, two weeks, three weeks, six weeks, eight weeks, ten weeks, 12 weeks, 24 weeks, 40 weeks, 60 weeks, 80 weeks, 100 weeks, 120 weeks, one week or more, two weeks or more, three weeks or more, six weeks or more, eight weeks or more, ten weeks or more, 12 weeks or more, 24 weeks or more, 40 weeks or more, 60 weeks or more, 80 weeks or more, 100 weeks or more, 120 weeks or more, six weeks or more and 24 weeks or less, 12 weeks or more and 24 weeks or less, six weeks or more and 30 weeks or less, 12 weeks or more and 30 weeks or less, six weeks or more and 40 weeks or less, 12 weeks or more and 40 weeks or less, six weeks or more and 60 weeks or less, 12 weeks or more and 60 weeks or less, six weeks or more and 80 weeks or less, 12 weeks or more and 80 weeks or less, six weeks or more and 100 weeks or less, 12 weeks or more and 100 weeks or less, six weeks or more and 120 weeks or less, or 12 weeks or more and 120 weeks or less.

In one embodiment, by continuously administering the pharmaceutical composition provided by the present invention for six weeks, 12 weeks, and/or 24 weeks, a beneficial effect (for example, an anemia-suppressing effect) can be detected in a kidney disease (for example, acute kidney disease or chronic kidney disease) patient.

In one embodiment, the pharmaceutical composition provided by the present invention is administered to a human suffering from kidney disease. Kidney disease includes acute kidney disease and chronic kidney disease, unless otherwise noted.

Examples of acute kidney disease include acute kidney disease caused by a drug (for example, a non-steroidal anti-inflammatory drug, an angiotensin converting enzyme inhibitor, an angiotensin II receptor blocker, an aminoglycoside-based antibiotic, a new quinolone-based antibacterial agent, an iodine contrast agent, or a platinum formulation such as cisplatin) and acute kidney disease caused by renal ischemia.

Chronic kidney disease (CKD) is a concept including chronic kidney disease regardless of a primary disease, and is a concept including all pathological conditions that a renal function represented by glomerular filtration rate (GFR) decreases or findings suggesting renal disorder persist chronically (three months or more).

According to the CKD Clinical Guide 2012 (Journal of the Japanese Renal Society 2012), the severity of chronic kidney disease is evaluated by classification with cause (Cause: C), renal function (GFR: G), and proteinuria (albuminuria: A).

The classification with GFR is as follows.
G1: GFR is normal or high (≥ 90 mL/min/1.73 m²)
G2: GFR is normal or slightly decreased (60 to 89 mL/min/1.73 m²)
G3a: GFR is slightly or moderately decreased (45 to 59 mL/min/1.73 m²)
G3b: GFR is moderately or severely decreased (30 to 44 mL/min/1.73 m²)
G4: GFR is severely decreased (15 to 29 mL/min/1.73 m²)
G5: End-stage kidney disease (ESKD) (< 15 mL/min/1.73 m²)

When a primary disease is diabetes, the classification with proteinuria (albuminuria: A) is performed as follows using a urinary albumin/creatinine (Cr) ratio.
A1: Normal (less than 30 mg/gCr)
A2: Microalbuminuria (30 to 299 mg/gCr)
A3: Macroalbuminuria (300 mg/gCr or more)

When a primary disease is hypertension, nephritis, polycystic kidney, transplanted kidney, or the like, other than diabetes, the classification with proteinuria (albuminuria: A) is performed as follows using the urinary protein/creatinine (Cr) ratio.
A1: Normal (less than 0.15 g/gCr)
A2: Slight proteinuria (0.15 to 0.49 g/gCr)
A3: Severe proteinuria (0.50 g/gCr or more)

According to the Clinical Practice Guidebook for Diagnosis and Treatment of CKD 2012 (Journal of the Japanese Society of Nephrology 2012), the severity classification of chronic kidney disease (CKD) is described as, for example, diabetes G2A3, chronic nephritis G3bA1, or the like, using the above alphabets C, G, and A.

However, it is considered that the severity of chronic kidney disease has been described only as stages classified with GFR in the past, and the severity of chronic kidney disease can also be described as stages of G1, G2, G3a, G3b, G4, and G5 as in the past.

In one embodiment, the pharmaceutical composition provided by the present invention is administered to an early-stage chronic kidney disease patient with low severity.

In one embodiment, the pharmaceutical composition provided by the present invention is administered to a chronic kidney disease patient at stage G3b or lower, preferably at stage G2 or lower.

In one embodiment, the pharmaceutical composition provided by the present invention is administered to a chronic kidney disease patient at stage G2 or higher and stage G3b or lower (for example, at stage G2 and stage G3a; or at stage G2, stage G3a, and stage G3b).

In one embodiment, the pharmaceutical composition provided by the present invention is administered to a chronic kidney disease patient at stage G3b or lower and with microalbuminuria, preferably to a chronic kidney disease patient at stage G2 and with microalbuminuria.

In one embodiment, the pharmaceutical composition provided by the present invention is administered to a chronic kidney disease patient at stage G2 or higher and stage G3b or lower (for example, at stage G2 and stage G3a; or at stage G2, stage G3a, and stage G3b) and with microalbuminuria.

In one embodiment, the pharmaceutical composition provided by the present invention is administered to a chronic kidney disease patient at stage G3b or lower and with urinary protein excretion of less than 3.5 g/gCr, preferably to a chronic kidney disease patient at stage G2 and with urinary protein excretion of less than 3.5 g/gCr.

In one embodiment, the pharmaceutical composition provided by the present invention is administered to a chronic kidney disease patient at stage G2 or higher and stage G3b or lower (for example, at stage G2 and stage G3a; or at stage G2, stage G3a, and stage G3b) and with urinary protein excretion of less than 3.5 g/gCr.

In one embodiment, the pharmaceutical composition provided by the present invention is administered to a progressive chronic kidney disease patient.

In one embodiment, the pharmaceutical composition provided by the present invention is administered to a chronic kidney disease patient with anemia.

In one embodiment, the pharmaceutical composition provided by the present invention is administered to a chronic kidney disease patient with hypertension.

In one embodiment, the pharmaceutical composition provided by the present invention is administered to a patient who receives treatment according to the Clinical Practice Guidebook for Diagnosis and Treatment of CKD. For example, the pharmaceutical composition provided by the present invention is administered to a patient who undergoes blood pressure control (for example, administration of an RA-based inhibitor such as ARB or an ACE inhibitor, diuretic, or a Ca antagonist), measures against proteinuria (for example, administration of an RA-based inhibitor), blood glucose level control (for example, administration of an a-glucosidase inhibitor), lipid control (for example, administration of statin or fibrate), anemia control (for example, administration of erythropoetin), and/or measures against bone and minerals (for example, administration of bisphosphonate) according to the Clinical Practice Guidebook for Diagnosis and Treatment of CKD.

In one embodiment, the pharmaceutical composition provided by the present invention is used in combination with an antihypertensive agent (for example an ARB, an ACE inhibitor, diuretics, or a Ca antagonist).

In one embodiment, the pharmaceutical composition provided by the present invention is used in combination with spherical adsorbed carbon obtained by oxidizing and reducing spherical fine porous carbon derived from a petroleum-based hydrocarbon at a high temperature (sold as Kremezin (registered trademark) in Japan).

In one embodiment, the pharmaceutical composition provided by the present invention is administered to an early-stage chronic kidney disease patient with low severity (for example, a chronic kidney disease patient at stage G3b or lower, preferably at stage G2 or higher and stage G3b or lower, more preferably at stage G2 and stage G3a, still more preferably at stage G2), and can suppress anemia in the patient. In this embodiment, the pharmaceutical composition provided by the present invention may be an acidosis-suppressing pharmaceutical composition or an anemia-suppressing pharmaceutical composition for a chronic kidney disease patient with anemia.

In one embodiment, the pharmaceutical composition provided by the present invention is administered to an early-stage chronic kidney disease patient with low severity (for example, a chronic kidney disease patient at stage G3b or lower, preferably at stage G2 or higher and stage G3b or lower, more preferably at stage G2 and stage G3a, still more preferably at stage G2), and suppresses a decrease in blood iron concentration or a decrease in blood ferritin concentration as compared with a case where sodium bicarbonate is administered. In this embodiment, the pharmaceutical composition provided by the present invention may be an acidosis-suppressing agent, an agent for suppressing a decrease in blood iron concentration, or an agent for suppressing a decrease in blood ferritin concentration for a chronic kidney disease patient with anemia.

In one embodiment, the pharmaceutical composition provided by the present invention is a pharmaceutical composition for use in suppressing a decrease in blood iron concentration or suppressing a decrease in blood ferritin concentration, and contains potassium citrate monohydrate and sodium citrate dihydrate as active ingredients, which are orally administered each in an amount of at 0.5 to 1.5 g/day for a total of 1 to 3 g/day, in which the daily dose is divided into one to five portions, preferably three portions, to be administered a day.

In one embodiment, the pharmaceutical composition provided by the present invention is a pharmaceutical composition for use in suppressing a decrease in blood iron concentration or suppressing a decrease in blood ferritin concentration, contains, in one administration unit (preferably one tablet), 231.5 mg of potassium citrate monohydrate and 195.0 mg of sodium citrate dihydrate, and is orally administered in three to six administration units a day (for example, three administration units or six administration units a day), in which the daily dose is divided into three portions to be administered a day.

In one embodiment, the pharmaceutical composition provided by the present invention is a pharmaceutical composition for use in suppressing a decrease in blood iron concentration or suppressing a decrease in blood ferritin concentration, contains, in one administration unit (preferably one tablet), 72.5 mg of anhydrous citric acid, 231.5 mg of potassium citrate monohydrate, and 195.0 mg of sodium citrate dihydrate, and is orally administered in three to six administration units a day (for example, three administration units or six administration units a day), in which the daily dose is divided into three portions to be administered a day.

In one embodiment, the pharmaceutical composition provided by the present invention is a pharmaceutical composition for use in suppressing a decrease in blood iron concentration or suppressing a decrease in blood ferritin concentration in a chronic kidney disease patient, and contains potassium citrate monohydrate and sodium citrate dihydrate as active ingredients, which are orally administered each in an amount of at 0.5 to 1.5 g/day for a total of 1 to 3 g/day, in which the daily dose is divided into one to five portions, preferably three portions, to be administered a day.

In one embodiment, the pharmaceutical composition provided by the present invention is a pharmaceutical composition for use in suppressing a decrease in blood iron concentration or suppressing a decrease in blood ferritin concentration in a chronic kidney disease patient, contains, in one administration unit (preferably one tablet), 231.5 mg of potassium citrate monohydrate and 195.0 mg of sodium citrate dihydrate, and is orally administered in three to six administration units a day (for example, three administration units or six administration units a day), in which the daily dose is divided into three portions to be administered a day.

In one embodiment, the pharmaceutical composition provided by the present invention is a pharmaceutical composition for use in suppressing a decrease in blood iron concentration or suppressing a decrease in blood ferritin concentration in a chronic kidney disease patient, contains, in one administration unit (preferably one tablet), 72.5 mg of anhydrous citric acid, 231.5 mg of potassium citrate monohydrate, and 195.0 mg of sodium citrate dihydrate, and is orally administered in three to six administration units a day (for example, three administration units or six administration units a day), in which the daily dose is divided into three portions to be administered a day.

In one embodiment, the pharmaceutical composition provided by the present invention is a pharmaceutical composition for use in suppressing acidosis or suppressing anemia in a chronic kidney disease patient with anemia, and contains potassium citrate monohydrate and sodium citrate dihydrate as active ingredients, which are orally administered each in an amount of at 0.5 to 1.5 g/day for a total of 1 to 3 g/day, in which the daily dose is divided into one to five portions, preferably three portions, to be administered a day.

In one embodiment, the pharmaceutical composition provided by the present invention is a pharmaceutical composition for use in suppressing acidosis or suppressing anemia in a chronic kidney disease patient with anemia, contains, in one administration unit (preferably one tablet), 231.5 mg of potassium citrate monohydrate and 195.0 mg of sodium citrate dihydrate, and is orally administered in three to six administration units a day (for example, three administration units or six administration units a day), in which the daily dose is divided into three portions to be administered a day.

In one embodiment, the pharmaceutical composition provided by the present invention is a pharmaceutical composition for use in suppressing acidosis or suppressing anemia in a chronic kidney disease patient with anemia, contains, in one administration unit (preferably one tablet), 72.5 mg of anhydrous citric acid, 231.5 mg of potassium citrate monohydrate, and 195.0 mg of sodium citrate dihydrate, and is orally administered in three to six administration units a day (for example, three administration units or six administration units a day), in which the daily dose is divided into three portions to be administered a day.

Examples of other embodiments of the present invention include the following:
a) a method for suppressing acidosis in chronic kidney disease or acute kidney disease with anemia in a mammalian subject (for example, a human), the method including administering an effective amount of citric acid, a pharmaceutically acceptable salt of citric acid, a hydrate thereof, or a mixture thereof to a subject in need of suppression of acidosis in chronic kidney disease or acute kidney disease with anemia;
b) a method for suppressing renal anemia or iron deficiency anemia in a mammalian subject (for example, a human), the method including administering an effective amount of citric acid, a pharmaceutically acceptable salt of citric acid, a hydrate thereof, or a mixture thereof to a subject in need of suppression of renal anemia or iron deficiency anemia;
c) a method for treating or preventing renal anemia or iron deficiency anemia in a mammalian subject (for example, a human), the method including administering an effective amount of citric acid, a pharmaceutically acceptable salt of citric acid, a hydrate thereof, or a mixture thereof to a subject in need of treatment or prevention of renal anemia or iron deficiency anemia;
aa) citric acid, a pharmaceutically acceptable salt of citric acid, a hydrate thereof, or a mixture thereof for use in suppressing acidosis in chronic kidney disease or acute kidney disease with anemia;
bb) citric acid, a pharmaceutically acceptable salt of citric acid, a hydrate thereof, or a mixture thereof for use in suppressing renal anemia or iron deficiency anemia;
cc) citric acid, a pharmaceutically acceptable salt of citric acid, a hydrate thereof, or a mixture thereof for use in treating or preventing renal anemia or iron deficiency anemia;
aaa) a pharmaceutical composition containing citric acid, a pharmaceutically acceptable salt of citric acid, a hydrate thereof, or a mixture thereof for use in suppressing acidosis in chronic kidney disease or acute kidney disease with anemia;
bbb) a pharmaceutical composition containing citric acid, a pharmaceutically acceptable salt of citric acid, a hydrate thereof, or a mixture thereof for use in suppressing renal anemia or iron deficiency anemia;
ccc) a pharmaceutical composition containing citric acid, a pharmaceutically acceptable salt of citric acid, a hydrate thereof, or a mixture thereof for use in treating or preventing renal anemia or iron deficiency anemia;
aaaa) use of citric acid, a pharmaceutically acceptable salt of citric acid, a hydrate thereof, or a mixture thereof for manufacturing a pharmaceutical composition for suppressing acidosis in chronic kidney disease or acute kidney disease with anemia;
bbbb) use of citric acid, a pharmaceutically acceptable salt of citric acid, a hydrate thereof, or a mixture thereof for manufacturing a pharmaceutical composition for suppressing renal anemia or iron deficiency anemia; and
cccc) use of citric acid, a pharmaceutically acceptable salt of citric acid, a hydrate thereof, or a mixture thereof for manufacturing a pharmaceutical composition for treating or preventing renal anemia or iron deficiency anemia;

### 2. Food composition

In one embodiment, a food composition provided by the present invention exhibits an effect of suppressing acidosis or suppressing anemia in a chronic kidney disease patient or an acute kidney disease patient with anemia (for example, suppressing renal anemia or suppressing iron deficiency anemia).

As the active ingredient, the active ingredient described in "1. Pharmaceutical composition" above can be applied. Examples of the active ingredient include a pharmaceutically acceptable salt of citric acid (for example, an alkali metal citrate, a hydrate thereof, or a mixture thereof) as a food-acceptable salt of citric acid, and preferable examples thereof include a mixture of potassium citrate monohydrate (C₆H₅K₃O₇•H₂O) and sodium citrate dihydrate (C₆H₅Na₃O₇•2H₂O), and sodium citrate dihydrate.

The content of citric acid, a food-acceptable salt of citric acid, a hydrate thereof, or a mixture thereof in the food composition provided by the present invention can be appropriately determined depending on the type of food. Examples of the food composition include a food for specified health use, a functionally labeled food, a food for hospital patients, and a supplement. A form of the food composition is not particularly limited, as long as the food composition contains citric acid, a food-acceptable salt of citric acid, a hydrate thereof, or a mixture thereof in an amount effective for exhibiting the above effect, and can be orally ingested. The food composition may be in a form of ordinary food or drink, or may be provided as a formulation suitable for oral administration such as a tablet, a capsule, or a suspending agent among formulations applicable to the pharmaceutical composition. Regarding the composition of the formulation and a method for manufacturing the formulation, here, the composition of the pharmaceutical formulation and a method for manufacturing the pharmaceutical formulation described in "1. Pharmaceutical composition" above can be applied as they are, and formulation technology that is itself known in the field of pharmaceutical formulation technology can be applied.

For example, a food for specified health use, a functionally labeled food, a food for hospital patients, or a supplement may contain potassium citrate monohydrate and sodium citrate dihydrate as active ingredients in total in an amount of 1/3 of 1 to 3 g per serving of food. When a food for specified health use, a functionally labeled food, a food for hospital patients, or a supplement is provided in a form of a tablet, for example, the tablet of 300 mg to 600 mg may contain 70 to 80% by weight of citric acid, a food-acceptable salt of citric acid, a hydrate thereof, or a mixture thereof.

When the food composition provided by the present invention is not formulated and is provided in a form of ordinary food or drink, the food composition can be appropriately manufactured by a person skilled in the art depending on the type of the food and, for example, can be manufactured by blending citric acid, a food-acceptable salt of citric acid, a hydrate thereof, or a mixture thereof (for example, potassium citrate and/or sodium citrate) with a food material.

Examples of a form of the food or drink include a liquid, milky, or pasty food such as a beverage, soy sauce, milk, yogurt, or soybean paste; a semi-solid food such as jelly or gummi; a solid food such as candy, gum, tofu, or a supplement; and a powdered food.

Examples of the beverage include a fruit juice/fruit beverage, a coffee beverage, an oolong tea beverage, a green tea beverage, a black tea beverage, a barley tea beverage, a vegetable beverage, a carbonated beverage that is a soft drink, a fruit extract-containing beverage, a vegetable extract-containing juice, near water, a sports beverage, and a diet beverage.

The beverage can contain additives such as an antioxidant, a fragrance, various esters, organic acids, organic acid salts, inorganic acids, inorganic acid salts, inorganic salts, pigments, an emulsifier, a preservative, a seasoning, a sweetener, an acidulant, fruit juice extracts, vegetable extracts, flower honey extracts, a pH regulator, and a quality stabilizer singly or in combination thereof.

The food composition provided by the present invention can be used in a similar manner to the method for using a pharmaceutical composition described in "1. Pharmaceutical composition" above, and can also be used in a range not intended to treat or prevent disease. That is, based on citric acid, a food-acceptable salt of citric acid, a hydrate thereof, or a mixture thereof contained in the food composition according to the present invention, the food composition provided by the present invention can be applied to an application subject of the pharmaceutical composition such that the use amount of citric acid, a food-acceptable salt of citric acid, a hydrate thereof, or a mixture thereof in the food composition is equal to the amount of citric acid, a pharmaceutically acceptable salt of citric acid, a hydrate thereof, or a mixture thereof contained in the pharmaceutical composition. In one embodiment, the "food composition" according to the present invention can be applied to a subject (for example, a human or another mammal) not having a "pathological" or "abnormal" symptom, condition, or disease, that is, a subject (for example, a human or another mammal) in a "healthy" or "normal" condition in order to maintain or promote a "healthy" or "normal" condition. Furthermore, the "food composition" according to the present invention can be applied to a "healthy person worrying about anemia" in order to maintain or promote a "healthy" or "normal" condition. In this case, even if the citric acid, food-acceptable salt of citric acid, hydrate thereof, or mixture thereof is an ingredient of a pharmaceutical composition or an ingredient of a food composition, a pharmacological effect of the citric acid, food-acceptable salt of citric acid, hydrate thereof, or mixture thereof itself is basically the same. Therefore, the application amount and application method of the food composition can be appropriately adjusted depending on an expected effect based on the citric acid, food-acceptable salt of citric acid, hydrate thereof, or mixture thereof.

The food composition applied to a subject (for example, a human or another mammal) not having a "pathological" or "abnormal" symptom, condition, or disease, that is, a subject (for example, a human or another mammal) in a "healthy" or "normal" condition in order to maintain or promote a "healthy" or "normal" condition may be particularly referred to as a "functionally labeled food".

The term "administration" described in "1. Pharmaceutical composition" above can also be applied to the "food composition" according to the present invention. Furthermore, regarding the "food composition" according to the present invention, the term "administration" can be read as "ingestion." Therefore, for example, the terms "administer", "administered", and the like, can be read as "ingest", "ingested", and the like, by changing the word form according to the context.

Therefore, examples of embodiments of the food composition according to the present invention include the following:

<1> an anemia-suppressing food composition containing citric acid, a food-acceptable salt of citric acid, a hydrate thereof, or a mixture thereof;
<11> a method for suppressing anemia, the method including allowing a subject in need of suppression of anemia to ingest a food composition containing an effective amount of citric acid, a food-acceptable salt of citric acid, a hydrate thereof, or a mixture thereof;
<111> use of a food composition containing citric acid, a food-acceptable salt of citric acid, a hydrate thereof, or a mixture thereof for suppressing anemia; and
<1111> use of citric acid, a food-acceptable salt of citric acid, a hydrate thereof, or a mixture thereof for manufacturing an anemia-suppressing food composition.

On a packaging, a container, or an instruction manual of the food composition according to the present invention, an effect of suppressing anemia, or the like, is preferably displayed.

Hereinafter, the present invention will be further described with reference to Examples, but the present invention is not limited thereto.

### Examples

A human clinical trial was performed in order to examine effects of oral administration of a potassium citrate/sodium citrate hydrate blending formulation and a baking soda formulation, which are oral alkalizing agents.

### 1. Method

47 chronic kidney disease patients at stage G2 to G3b (eGFR: 30 to 89 ml/min/1.73 m²) were randomly divided into a potassium citrate/sodium citrate hydrate blending formulation administration group (group A: 16 patients), a baking soda (sodium bicarbonate) formulation administration group (group B: 16 patients), and a control group (group C: 15 patients). Patients were assigned to the groups such that age, sex, presence or absence of diabetes, and eGFR were not biased. Each of the groups received treatment based on the "Clinical Practice Guidebook for Diagnosis and Treatment of CKD-Summary of Treatment" (hereinafter referred to as standard treatment).

No alkalizing agent was administered to the control group. To group A, three tablets each containing 231.5 mg of potassium citrate monohydrate (C₆H₅K₃O₇•H₂O) and 195.0 mg of sodium citrate hydrate (C₆H₅Na₃O₇•2H₂O) were orally administered a day for 24 weeks, in which the daily dose was divided into three portions (morning, noon, and evening). Note that the pH of early morning urine was controlled over time, and in cases where early morning urine had a pH of less than 6.5, the daily dose could be appropriately increased up to six tablets at discretion of a doctor, in which the daily dose was divided into three portions (morning, noon, and evening). To group B, three tablets each containing 500 mg of sodium bicarbonate were orally administered a day for 24 weeks, in which the daily dose was divided into three portions (morning, noon, and evening). Note that the pH of early morning urine was controlled over time, and in cases where early morning urine had a pH of less than 6.5, the daily dose could be appropriately increased up to six tablets at discretion of a doctor, in which the daily dose was divided into three portions (morning, noon, and evening).

Early morning urine, spot urine, and blood were collected before start of administration and 6 weeks, 12 weeks, and 24 weeks after start of administration, and each sample was stored at -80°C.

A serum iron concentration was measured by a nitroso-PSAP method, and a serum ferritin concentration was measured by a CLEIA (chemiluminescent enzyme immunoassay) method.

For statistical analysis, Mann-Whitney test was used for comparison between groups, and Wilcoxon test was used for comparison in a change over time. Pearson test was used for correlation.

### 2. Result

From the measurement results, the following was calculated for each patient in group A (potassium citrate/sodium citrate hydrate blending formulation administration group), group B (sodium bicarbonate formulation administration group), and group C (control group):

(i) Serum iron concentration and serum ferritin concentration before start of administration
(ii) Serum iron concentration 6 weeks, 12 weeks, and 24 weeks after start of administration
(iii) Serum ferritin concentration 24 weeks after start of administration
(iv) Ratio between ferritin concentration 24 weeks after start of administration and serum ferritin concentration before start of administration (serum ferritin concentration 24 weeks after start of administration/serum ferritin concentration before start of administration)
(viii) Amount of change in serum ferritin concentration 24 weeks after start of administration from serum ferritin concentration before start of administration

Then, for the above (i) to (iv), an average value and SD in each group were calculated.

Results thereof are illustrated in Tables below. Note that in Tables and the drawing, group A: potassium citrate/sodium citrate hydrate blending formulation administration group was described as "Citrate", and group B: sodium bicarbonate formulation administration group was described as "Bicarbonate".

Table 1: Serum iron amount (µg/dL),
Table 2: Serum ferritin concentrations (ng/mL) before start of administration and 24 weeks after start of administration; Ratio (%) between serum ferritin concentration 24 weeks after start of administration and serum ferritin concentration before start of administration; and Amount of change (ng/mL) in serum ferritin concentration from serum ferritin concentration before start of administration

### [Table 1]

**Table 1 Serum Fe (µg/dL)**

| Group | N | 0W | 6W | 12W | 24W | 6-24W^{c}(81-91) |
|---|---|---|---|---|---|---|
| Control | 30-31 | 89.6 ± 27.5 | 95.1 ± 30.0 | 90.6 ± 37.3 | 91.3 ± 25.7 | 92.3 ± 31.2^{b} |
| Citrate | 27-31 | 95.8 ± 29.2 | 102.2 ± 33.0 | 101.0 ± 34.5 | 93.6 ± 32.8 | 99.8 ± 33.2 |
| Bicarbonate | 26-30 | 95.2 ± 30.7 | 90.7 ± 23.5 | 90.0 ± 36.9 | 87.0 ± 30.9 | 89.2 ± 30.7^{a,d} |

| | | | | | | |
|---|---|---|---|---|---|---|
| Mean +/- SD ^{a}p=0.0323 and ^{b}p=0.1015 vs Uralyt (Mann-Whitney) Not Significant vs 0 week (Wilcoxon) ^{c}p=0.0799 (Kruskal-Wallis) and ^{d}p=0.0936 va Uralvt (Dunn) (There is no significant difference between groups of 0 week) | | | | | | |

In group B (Bicarbonate: sodium bicarbonate formulation administration group), the serum iron concentrations 6 to 24 weeks after start of administration were significantly decreased, as compared with those in group A (Citrate: potassium citrate/sodium citrate hydrate blending formulation administration group). In group A (Citrate: potassium citrate/sodium citrate hydrate blending formulation administration group) and group C (Control: control group), the serum iron concentrations 6 to 24 weeks after start of administration were not decreased (see Table 1). Therefore, this suggests that administration of the potassium citrate/sodium citrate hydrate blending formulation suppresses anemia, as compared with administration of the sodium bicarbonate formulation. Both the potassium citrate/sodium citrate hydrate blending formulation and the sodium bicarbonate formulation are used for treating acidosis in chronic kidney disease. The above results suggest that the potassium citrate/sodium citrate hydrate blending formulation can be used suitably for treating acidosis without causing anemia, as compared with the sodium bicarbonate formulation.

### [Table 2]

**Table 2 Serum Ferritin**

| Group | N | 0W (ng/mL) | 24W | | |
|---|---|---|---|---|---|
| | | | ng/mL | 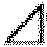 % | 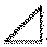 ng/mL |
| Control | 27 | 103.2 ± 93.6 | 90.7 ± 83.8^{b} | 94 ± 31 | - 12.5 ± 28.7 |
| Citrate | 27 | 135.4 * 109.9 | 124.9 ± 92.5 | 100 ± 34^{a} | - 10.5 ± 43.2 |
| Bicarbonate | 27 | 119.7 + 132.7 | 99.8 ± 100.7^{c} | 85 ± 23 | - 19.9 ± 49.0 |

| | | | | | |
|---|---|---|---|---|---|
| Mean +/- SD ^{a}p=0.0697 vs Bicarbonate (Mann-Whitney) ^{b}p=0.0525 and ^{c}p=0.0004 vs 0 week (Wilcoxon) (There is no significant difference between groups of 0 week) | | | | | |

In group A (Citrate: potassium citrate/sodium citrate hydrate blending formulation administration group) and group C (Control: control group), the serum ferritin concentrations tended to decrease 24 weeks after start of administration, as compared with those 0 week after start of administration. Meanwhile, in group B (Bicarbonate: sodium bicarbonate formulation administration group), the serum ferritin concentration was significantly decreased 24 weeks after start of administration as compared with that 0 week after start of administration. The decrease ratio in serum ferritin concentration 24 weeks after start of administration as compared with that 0 week after start of administration was significantly larger in group B (Bicarbonate: sodium bicarbonate formulation administration group), as compared with group A (Citrate: potassium citrate/sodium citrate hydrate blending formulation administration group) (see Table 2). Therefore, this suggests that administration of the potassium citrate/sodium citrate hydrate blending formulation suppresses anemia, as compared with administration of the sodium bicarbonate formulation. Both the potassium citrate/sodium citrate hydrate blending formulation and the sodium bicarbonate formulation are used for treating acidosis in chronic kidney disease. The above results suggest that the potassium citrate/sodium citrate hydrate blending formulation can be used suitably for treating acidosis without causing anemia, as compared with the sodium bicarbonate formulation.

### Industrial Applicability

The pharmaceutical composition, and the like, provided by the present invention can suppress acidosis or anemia in a chronic kidney disease patient with anemia in a mammal.

## Claims

1. A pharmaceutical composition for treating or preventing acidosis in chronic kidney disease with anemia, the pharmaceutical composition comprising citric acid, a pharmaceutically acceptable salt of citric acid, a hydrate thereof, or a mixture thereof.

2. The pharmaceutical composition according to claim 1, wherein citric acid, a pharmaceutically acceptable salt of citric acid, a hydrate thereof, or a mixture thereof is administered in an amount of 1 to 3 g/day.

3. The pharmaceutical composition according to claim 1 or 2, wherein citric acid, a pharmaceutically acceptable salt of citric acid, a hydrate thereof, or a mixture thereof is administered in an amount of 1 to 1.5 g/day.
